# EUROPEAN PATENT SPECIFICATION

(11) **EP 2 852 437 B1**
(45) Date of publication and mention of the grant of the patent: **06.04.2016**
(21) Application number: 13728982.3
(22) Date of filing: 22.05.2013
(51) Int. Cl.: A61P 19/02, A61P 19/00, A61K 31/726, A61K 31/737, A61K 45/06, A61K 31/375, C08B 37/00

(54) **LOW-MOLECULAR-WEIGHT BIOTECHNOLOGICAL CHONDROITIN 6-SULPHATE FOR PREVENTION OF OSTEOARTHRITIS**
NIEDERMOLEKULARES BIOTECHNOLOGISCHES CHONDROITIN-6-SULFAT ZUR PRÄVENTION VON OSTEOARTHRITIS
6-SULFATE DE CHONDROÏTINE BIOTECHNOLOGIQUE DE FAIBLE MASSE MOLÉCULAIRE POUR LA PRÉVENTION DE L'ARTHROSE

(30) Priority: 22.05.2012 IT MI20120880
(43) Date of publication of application: 01.04.2015
(73) Proprietor: Gnosis S.p.A., 20121 Milano (IT)
(72) Inventor: MIRAGLIA, Niccolò, I-20033 Desio (MB) (IT); BIANCHI, Davide, I-20033 Desio (MB) (IT); VALOTI, Ermanno, I-24044 Dalmine (BG) (IT); TRENTIN, Antonella, I-20033 Desio (MB) (IT); TRILLI, Antonio, I-20033 Desio (MB) (IT); BUSIELLO, Immacolata, I-20033 Desio (MB) (IT); AGOSTINETTO, Marco, I-20033 Desio (MB) (IT); BAZZA, Paola, I-20033 Desio (MB) (IT); VALETTI, Marco, I-20033 Desio (MB) (IT)
(74) Representative: Minoja, Fabrizio
(86) International application number: PCT/EP2013/060471
(87) International publication number: WO 2013/174847

(56) References cited:
- WO-A2-2005/110438
- CHO SO YEAN ET AL: "Effects of low molecular weight chondroitin sulfate on type II collagen-induced arthritis in DBA/1J mice", BIOLOGICAL & PHARMACEUTICAL BULLETIN (OF JAPAN), PHARMACEUTICAL SOCIETY OF JAPAN, TOKYO, JP , vol. 27, no. 1 1 January 2004 (2004-01-01), pages 47-51, XP008159629, ISSN: 0918-6158 Retrieved from the Internet: URL:http://www.jstage.jst.go.jp/browse/bpb cited in the application
- VOLPI N: "Influence of charge density, sulfate group position and molecular mass on adsorption of chondroitin sulfate onto coral", BIOMATERIALS, ELSEVIER SCIENCE PUBLISHERS BV., BARKING, GB, vol. 23, no. 14, 1 July 2002 (2002-07-01), pages 3015-3022, XP004353901, ISSN: 0142-9612, DOI: 10.1016/S0142-9612(02)00060-1 cited in the application
- TSUTSUMI K ET AL: "Functional expression and genomic structure of human chondroitin 6-sulfotransferase", FEBS LETTERS, ELSEVIER, AMSTERDAM, NL, vol. 441, no. 2, 18 December 1998 (1998-12-18), pages 235-241, XP004258907, ISSN: 0014-5793, DOI: 10.1016/S0014-5793(98)01532-4
- RODRIGUEZ M L ET AL: "STRUCTURE AND SEROLOGICAL CHARACTERISTICS OF THE CAPSULAR K4 ANTIGEN OF ESCHERICHIA COLI O5:K4:H4, A FRUCTOSE-CONTAINING POLYSACCAHRIDE WITH A CHONDROITIN BACKBONE", EUROPEAN JOURNAL OF BIOCHEMISTRY, BLACKWELL PUBLISHING, BERLIN, DE, vol. 177, no. 1, 1 January 1988 (1988-01-01), pages 117-124, XP000886039, ISSN: 0014-2956, DOI: 10.1111/J.1432-1033.1988.TB14351.X cited in the application

## Description

### Summary of the invention

The present invention relates to a chondroitin sulphate (CS) with an extremely low molecular weight (1000-5000 daltons) produced by chemical sulphation and subsequent depolymerisation of a non-sulphated chondroitin backbone obtained with biotechnology techniques, or produced by sulphation of a polysaccharide of biotechnological origin originally characterised by low molecular weight, and the use of said CS in the treatment and prevention of osteoarthritis and acute and chronic inflammatory processes. In particular, the invention relates to a biotechnological CS which is substantially monosulphated, mainly at the 6-position, possesses little or no 4-sulphate, and is identical to natural CS in terms of the mono/disulphated disaccharide ratio, the absence of tri-sulphated and polysulphated disaccharides, the charge density and the biological activity exhibited. The chondroitin 6-sulphate (C6S) according to the invention presents a lower molecular weight (1000-5000 daltons) than chondroitin sulphates extracted from animal tissues of terrestrial origin (bovine, porcine and avian), characterised by molecular weight values of 14,000-26,000 daltons, and of marine origin (shark, squid, skate and bony fish), all with a molecular weight > 50,000 daltons. The C6S according to the invention also has a molecular weight even lower than known types of low-molecular-weight CS. This characteristic gives the chondroitin 6-sulphate according to the invention better bioavailability and consequently greater efficiency.

The use of low-molecular-weight biotechnological chondroitin 6-sulphate (C6S) in the treatment and prevention of osteoarthritis is supported by the experimental verification of its anti-inflammatory activity in a well-known animal model normally used for the study of arthritis and the associated symptoms. The low-molecular-weight biotechnological C6S described also exhibits good tolerance, as demonstrated in toxicological studies conducted in accordance with the OECD guidelines for pharmaceutical products.

### Background to the invention

Chondroitin sulphate (CS) is currently recommended by EULAR (the European League against Rheumatism) as a symptomatic slow-acting drug for osteoarthritis (SYSADOA) in the treatment of osteoarthritis of the knee (Jordan KM et al., Ann. Rheum. Dis. 62, 1145, 2003), hip (Jordan KM et al. Ann. Rheum. Dis. 62, 1145, 2003) and hand (Zhang W et al., Ann. Rheum. Dis. 66, 377, 2007) on the basis of numerous clinical findings and various meta-analyses of clinical trials. Recent clinical trials have also demonstrated that CS modifies the extracellular structures of human cartilage tissue (Reginster JY, Heraud F, Zegels B, Bruyere O. Mini Rev Med Chem 7, 1051, 2007. Kahan A, Uebelhart D, De Vathaire F, Delmas PD, Reginster JY. Arthritis Rheum 60, 524, 2009). CS is also widely used as a nutraceutical, either alone or combined with other ingredients (McAlindon TE et al., JAMA 283, 1469, 2000. Volpi N et al., Food Anal Meth 1, 195, 2008. Volpi N et al., Separation Sc 1, 22, 2009).

Chondroitin sulphate (CS) is a natural polysaccharide belonging to the glycosaminoglycan (GAG) class, present in both vertebrates and invertebrates, which consists of disaccharide sequences formed by alternating residues of glucuronic acid (GlcA) and N-acetyl-D-galactosamine (GalNAc) bonded to one another by beta 1-3 bonds and sulphated in different positions.

CS is present in animal tissues, with structural and physiological functions. It mainly consists of two types of disaccharide unit monosulphated at the 4- or 6-position of GalNAc (called disaccharides A and C respectively), present in different percentages depending on its origin. The CS backbone also contains non-sulphated disaccharide, generally in small amounts. Disulphated disaccharides having two sulphate groups bonded through the oxygen atom at various positions, such as the 2-position of GlcA and the 6-position of GalNAc (disaccharide D), the 2-position of GlcA and the 4-position of GalNac, or the 4- and 6-positions of GalNAc (disaccharide E), can be present in the CS backbone in variable percentages, depending on the specific animal sources (Volpi N. J. Pharm. Pharmacol. 61, 1271, 2009. Volpi N. J. Pharm. Sci. 96, 3168, 2007. Volpi N. Curr. Pharm. Des. 12, 639, 2006). The presence of sulphation at the 3-position of GlcA is possible, but in extremely small amounts; said presence is rare in CS of terrestrial origin, and more probable in the highly sulphated types of marine origin (Fongmoon D et al. J Biol Chem 282, 36895, 2007).

The formula of the repeating disaccharide unit of CS is as follows: 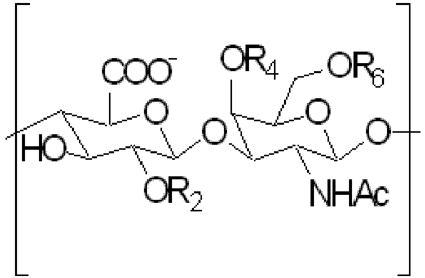
wherein R₂, R₄ and R₆ are independently H or SO₃⁻.

The negative charges of the carboxylate and sulphate groups in the repeating disaccharide unit are generally neutralised by sodium ions.

The meanings of the acronyms most commonly used to identify the variously sulphated disaccharides are set out below:
- Di-0S: (R2=H; R4=H; R6=H)
- Di-6S (C): (R2=H; R4=H; R6= SO3-)
- Di-4S (A): (R2=H; R4= SO3-; R6=H)
- Di-4,6diS (E): (R2=H; R4= SO3-; R6= SO3-)
- Di-2,6diS (D): (R2= SO3-; R4=H; R6= SO3-)
- Di-2,4diS (B): (R2= SO3-; R4= SO3-; R6=H)
- Di-2,4,6triS: (R2= SO3-; R4= SO3-; R6= SO3-)

Samples of CS originating from different animal sources are also characterised by different molecular weights and charge densities, this latter parameter being directly correlated with the specific sulphated groups.

Table 1 shows the main disaccharides found in natural CS extracted from cartilage of various animal species:

**Table 1 -**

| Parameters | Bovine CS | Porcine CS | Chicken CS | Shark CS | Skate CS | Squid CS |
|---|---|---|---|---|---|---|
| | | | | | | |
| Mn (kDa) | 12-17 | 9-14 | 8-13 | 25-40 | 27-34 | 60-80 |
| Mw (kDa) | 20-26 | 14-20 | 16-21 | 50-70 | 50-70 | 80-120 |
| Polydispersity index | 1.8-2.2 | 1.4-1.8 | 1.6-2.0 | 1.0-2.0 | 1.2-2.5 | 0.8-1.3 |
| | | | | | | |
| Di-0S | 6 | 6 | 8 | 3 | 3 | 13 |
| Di-6S | 33 | 14 | 20 | 44 | 39 | 15 |
| Di-4S | 61 | 80 | 72 | 32 | 43 | 50 |
| Di-2,6diS | ND | ND | ND | 18 | 13 | 0 |
| Di-4,6diS | ND | ND | ND | 2 | 1 | 22 |
| Di-2,4diS | ND | ND | ND | 1 | 1 | 0 |
| | | | | | | |
| Charge density | 0.90-0.96 | 0.92-0.96 | 0.90-0.94 | 1.15-1.25 | 1.08-1.20 | 1.00-1.20 |
| 4S/6S ratio | 1.50-2.00 | 4.50-7.00 | 3.00-4.00 | 0.45-0.90 | 1.00-1.40 | 2.50-4.00 |

| | | | | | | |
|---|---|---|---|---|---|---|
| Mn = number average molecular weight; Mw = weight average molecular weight; polydispersity index = Mw/Mn; the charge density is the number of sulphate groups per disaccharide unit; ND = not identified | | | | | | |

The various types of CS derived from terrestrial animals have similar molecular mass parameters (Mn and Mw), whereas they differ from those of marine species, which have higher molecular mass values. CS of terrestrial origin has a mean molecular weight (Mw) between 14 and 26 kDa, whereas CS of marine origin, obtained from squid, cartilaginous fish and bony fish, has a molecular weight (Mw) exceeding 50 kDa. Terrestrial CS samples are also characterised by charge density (CD) values below 1.0, whereas marine CS samples always have CD values exceeding 1.0.

Disulphated disaccharides are usually present in traces in terrestrial CS, and more abundant in CS of marine origin. Moreover, significant amounts of polysulphated disaccharides (tri- and tetra-sulphates) are not observed in natural CS.

Natural CS also presents differences between different organs and tissues, even in the same species, as shown in Table 2.

**Table 2 -**

| Parameters | Bovine cartilage | Bovine aorta | Sturgeon bones | Rabbit ileum, kidney, lung and bone marrow | Human platelets | Human plasma |
|---|---|---|---|---|---|---|
| | | | | | | |
| Mn (kDa) | 12-17 | ND | 25-30 | ND | ND | ND |
| Mw (kDa) | 20-26 | ND | 35-40 | ND | ND | ∼15 |
| Polydispersity index | 1.8-2.2 | ND | 1.05-1.5 | ND | ND | ND |
| | | | | | | |
| Di-0S | 6 | 0 | 7 | ND | 0 | 40-60 |
| Di-6S | 33 | 95-100 | 55 | ∼ 100 | Traces | 1-5 |
| Di-4S | 61 | 0-5 | 38 | Traces | > 98 | 60-40 |
| Di-2,6diS | ND | 0 | 0 | 0 | 0 | 0 |
| Di-4,6diS | ND | 0 | 0 | 0 | 0 | 0 |
| Di-2,4diS | ND | 0 | 0 | 0 | 0 | 0 |
| | | | | | | |
| Charge density | 0.90-0.96 | 0.98-1.02 | 0.90-0.95 | 0.98-1.02 | 0.98-1.02 | 0.40-0.60 |
| 4S/6S ratio | 1.50-2.00 | < 0.1 | 0.40-0.90 | < 0.1 | > 45 | 10-50 |

| | | | | | | |
|---|---|---|---|---|---|---|
| Mn = number average molecular weight; Mw = weight average molecular weight; polydispersity index = Mw/Mn; the charge density is the number of sulphate groups per disaccharide unit; ND = not identified. | | | | | | |

The existence of chains of polysaccharide or oligosaccharide CS with 100% 6-sulphate or 4-sulphate disaccharides is reported in the literature for various tissues and organs (Sampaio L.O. et al. Biol. Chem. 256, 9205, 1981; Okayama E. et al. Blood 72,745, 1988; Ambrosius M. et al. J. Chrom. A 1201, 54, 2008; Volpi N. et al. Clin. Chim. Acta 370, 196, 2006).

All these characteristics demonstrate the extreme heterogeneity of natural CS in terms of both molecular weight and charge density; however, parameters according to which a CS can be defined as "natural-like" can be identified. A chondroitin 6-sulphate which has a charge density comparable to that of CS of marine origin and is characterised by the absence of abnormal sulphation patterns presents as structurally similar to natural glycosaminoglycan. Its proven anti-inflammatory activity *in vivo* provides further support for the definition of natural-like CS, and supports its use in the treatment of symptoms correlated with arthritic disorders.

Many attempts have been made to find a biotechnological method for the production of CS using micro-organisms as a polysaccharide precursor source having a structure partly similar to that of CS, and then using chemical sulphation to produce a CS similar to the natural type.

Some bacteria produce capsular polysaccharides with a structure similar to glycosaminoglycans; for example, *Pasteurella multocida* produces a polysaccharide identical to non-sulphated chondroitin (De Angelis P.L., Carbohydrate Res., 337 (17), 1547, 2002). However, the *Escherichia coli* strain with serotype 05:K4:H4 produces a capsular polysaccharide with a chondroitin backbone bearing a β-fructose residue bonded at the 3-position of the GlcA unit (polysaccharide K4).

An example of production of biotechnological CS starting with capsular polysaccharide K4 from *E. coli* 05:K4:H4 is reported in EP 1304338, which describes a process wherein polysaccharide K4, produced in liquid cultures, is extracted and purified and then redissolved and subjected to acid hydrolysis to eliminate the fructose residues bonded to the GlcA residues of the polymer. The defructosylated polymer, identical to the non-sulphated backbone of CS (CH), is then sulphated at the 4- or 6-position of the GalNAc residue according to various chemical synthesis methods, to produce a CS with a molecular weight between 6 and 25 kDa. However, the biotechnological CS described in EP 1304338 is not evaluated at all for its anti-inflammatory and anti-arthritis activity, and its use in the treatment of arthritis and/or osteoarthritis remains a mere hypothesis. This is particularly important as only 70% of the polysaccharide described in EP 1304338 definitely has the structure of a natural chondroitin sulphate, the remaining 30% being mainly non-sulphated chondroitin (CH). Furthermore, oligosaccharides with a molecular weight of less than 5 kDa are not considered.

A recent publication (Bedini E. et al. Angew Chem. Int. Ed Engl. 2011) describes a process wherein the polysaccharide K4 produced is sulphated at the 4-position and/or the 6-position of the GalNAc residue in the same chain. Once again, the biotechnological CS described is not evaluated for anti-inflammatory or anti-arthritis activity, and its use in the treatment and prevention of arthritis and/or osteoarthritis and the correlated inflammatory processes is not evaluated. The same authors postulate the presence of structural modifications to the chain of biotechnological CS deriving from their synthesis process, which produces abnormal sulphation of the hydroxyl group in C3 of GlcA due to the low protection of that group during the synthesis process. This anomaly is known to cause serious toxicity in humans following intravenous administration of heparin wherein said CS 3-sulphated in GlcA was present as a contaminant. Although this toxicity has never been observed in relation to oral administration of CS, the risk of toxic effects due to that type of anomalous sulphation remains; this is also indicated by the same authors in another recent publication (Bedini E. et al. Chem. J. 2012 [Epub ahead of print]).

Moreover, the biotechnological CS described by Bedini et al. (Angew Chem Int Ed Engl. 2011) has a molecular weight of around 17 kDa, and therefore potentially exhibits the low bioavailability of natural products of extraction origin. For all these reasons, the biotechnological CS described by Bedini et al. is unlikely to be used in the treatment and prevention of arthritis and/or osteoarthritis.

Examples of low-molecular-weight types of CS for use in the treatment of arthritis do exist (Cho SY et al. Biol. Pharm. Bull. 27, 47, 2004, Das A. et al. Osteoart. Cartil. 8, 343, 2000), but they are all obtained by depolymerisation of CS of animal origin, which means that the presence of viruses, prions and other transmissible infectious agents cannot be ruled out.

### Brief description of the drawings

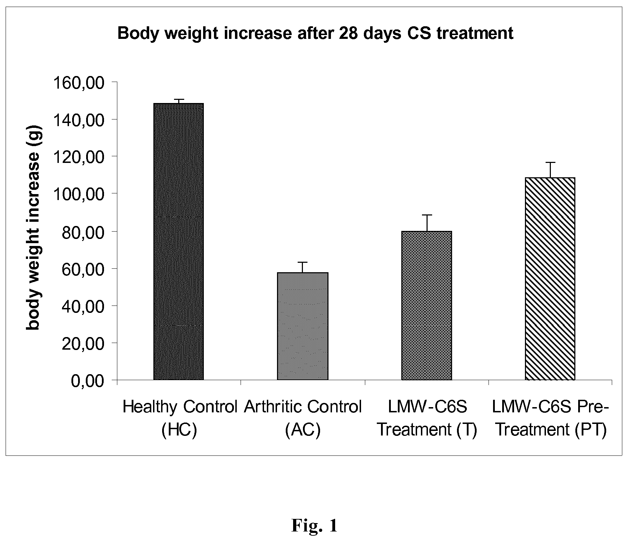

**Fig. 1****:** Increase in body weight of rats suffering from Adjuvant Arthritis (AA) following treatment with low-molecular-weight biotechnological C6S. Key: HC, healthy control; AC, arthritic control; T, group treated with C6S (days 0 to 28); PT, group pre-treated with C6S (days - 14 to 28). Values expressed in g ± SEM.

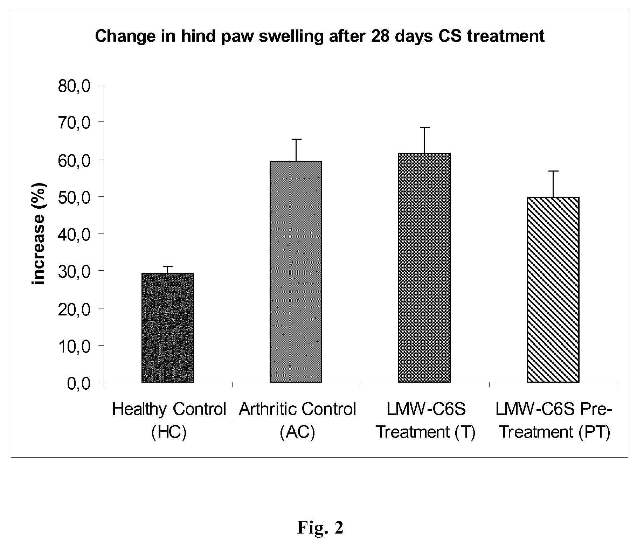

**Fig. 2****:** Evaluation of oedema in the hind limbs of rats suffering from Adjuvant Arthritis (AA) following treatment with low-molecular-weight biotechnological C6S. Key: HC, healthy control; AC, arthritic control; T, group treated with C6S (days 0 - 28); PT, group pre-treated with C6S (days -14 to 28). Percentage increase: measurement effected as increase in volume (ml), calculation of percentage: [(Dayₙ/Day₀) x 100] - 100 Values expressed as % ± SEM.

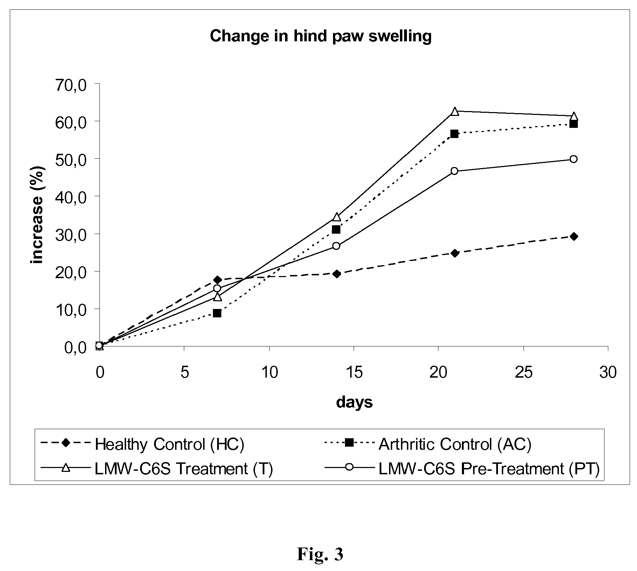

**Fig. 3****:** Progression of oedematous state during study in rats suffering from Adjuvant Arthritis (AA) following treatment with low-molecular-weight biotechnological C6S. Key: HC, healthy control; AC, arthritic control; T, group treated with C6S (days 0 - 28); PT, group pre-treated with C6S (days -14 to 28). Evaluation of percentage increase in volume 0, 7, 14, 21 and 28 days after induction of AA. Values expressed as %.

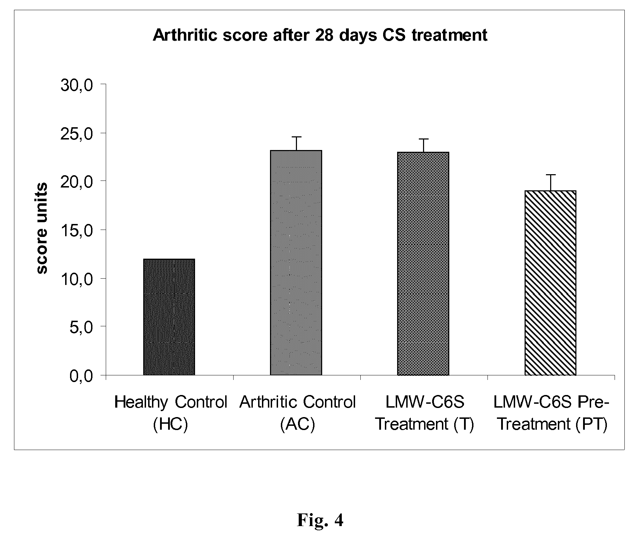

**Fig. 4****:** Arthritis score in rats suffering from Adjuvant Arthritis (AA) following treatment with low-molecular-weight biotechnological C6S. Key: HC, healthy control; AC, arthritic control; T, group treated with C6S (days 0 - 28); PT, group pre-treated with C6S (days -14 to 28). Score: periarticular swelling and erythema of forepaws (1 - 5), periarticular swelling and erythema of hind paws (1 - 8), diameter of scab at base of tail (1 - 5). Values expressed in units ± SEM.

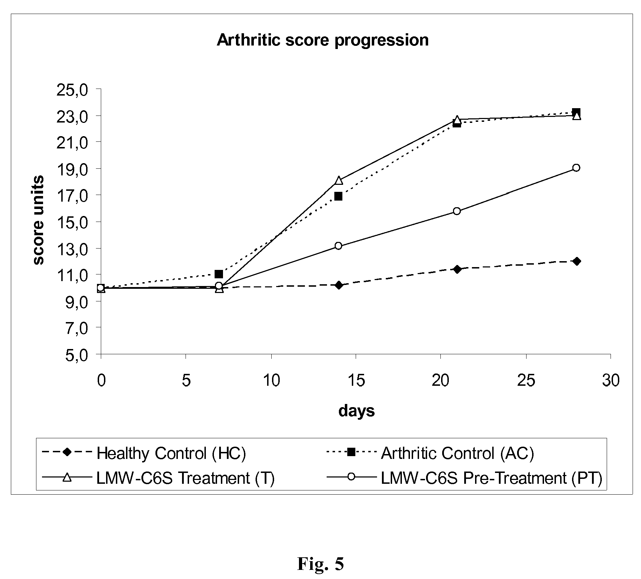

**Fig. 5****:** Progression of arthritis score during study in rats suffering from Adjuvant Arthritis (AA) following treatment with low-molecular-weight biotechnological C6S. Key: HC, healthy control; AC, arthritic control; T, group treated with C6S (days 0 - 28); PT, group pre-treated with C6S (days -14 to 28). Evaluation of score 0, 7, 14, 21 and 28 days after induction of AA. Values expressed in units.

### Description of the invention

It has now been found that a chondroitin sulphate (CS) with a low molecular weight, between 1000 and 5000 daltons, or preferably between 2000 and 4000 daltons, produced by chemical sulphation and subsequent depolymerisation of a non-sulphated chondroitin backbone obtained by biotechnological techniques, has an anti-inflammatory activity comparable with that of natural CS, improved bioavailability and a favourable safety profile. The CS described is substantially monosulphated, mainly at the 6-position, with very little sulphation at the 4-position, and with a mono/disulphated disaccharide ratio and charge density similar to those of natural CS.

The CS according to the invention presents all the characteristics of a natural CS, and more specifically of CS of marine origin. It has similar relative percentages of mono- and di-sulphated disaccharides, similar distribution of disulphated disaccharides and consequently a similar charge density (CD) associated with a low 4-sulphate/6-sulphate ratio.

The biotechnological CS according to the invention also has the following special characteristics: a very low molecular weight (between 1000 and 5000 daltons, or preferably between 2000 and 4000 daltons); a particularly high percentage of 6-sulphated disaccharides; an almost total absence of tri-sulphated disaccharides; substantial absence of sulphation at the 3-position of the GlcA residue. In particular, the presence of tri-sulphated disaccharides and disaccharides sulphated at the 3-position of GlcA characterises the known types of synthetic CS, and often causes adverse effects in their therapeutic application.

Table 3 shows the physicochemical characteristics of the biotechnological chondroitin 6-sulphate according to the invention.

**Table 3**

| Physicochemical characteristics of biotechnological CS | |
|---|---|
| Molecular mass (MWw) | 1000 - 5000 Da |
| *Disaccharides:* | |
| Δ Di-0S | < 15% |
| Δ Di-6S | ≥ 65% |
| Δ Di-4S | < 1% |
| Δ Di-2,6diS | < 20% |
| Δ Di-4,6diS | < 5% |
| Δ Di-2,4diS | < 1% |
| Charge Density | 1 - 1.25 |
| 4S / 6S ratio | < 0.1 |

According to a particular aspect of the invention, C6S can be obtained by the chemical synthesis process described in PCT/EP2011/058297 applied to the capsular polysaccharide K4 produced naturally from the *E. coli* strain O5:K4:H4 (WO 01/02597) previously defructosylated by thermoacid hydrolysis according to known techniques (Rodriguez and Jann, Eur.J.Biochem. 117, 117-124, FEBS 1988) or to any other polysaccharide with the structure of non-sulphated chondroitin. Alternatively, the starting non-sulphated chondroitin (CH) can be obtained from cultures of the *E. coli* strain *DSM23644* described in WO 2012004063 which, due to a mutation induced in the KfoE gene responsible for the fructosylation of K4, produces a polysaccharide identical to natural non-sulphated CH. According to this aspect of the invention, the polysaccharide undergoes chemical sulphation, preferably according to the method described in PCT/EP2011/058297.

Briefly, the synthesis process that leads to sulphation of the disaccharide units is as follows:
a) The unsulphated chondroitin, isolated as ammonium salt, or as any of the alkaline metal salts and particularly as sodium salt, or as potassium salt, or lithium salt obtained upon defructosylation of polysaccharide K4 is desalified on cation-exchange resin and resalified with an alkylammonium hydroxide group, preferably with tetrabutylammonium hydroxide added in a stoichiometric amount up to a pH of 7 - 7.5, and dried by freeze-drying or spray-drying.
b) The tetrabutylammonium CH salt described in step a) is added under stirring to a solution consisting of a polar aprotic solvent, preferably dimethylformamide (DMF), maintained at a temperature between 0 and 30°C; the sulphating complex is then added in a molar ratio between 2 and 5 to the CH, maintaining a constant temperature and stirring.
c) Finally, an amount of sodium bicarbonate is added in a stoichiometric molar ratio to the sulphating agent or in excess, at the same time increasing the temperature to 65°C to evaporate off the solvent. Water is then added, followed by redistillation. The final solution is ultrafiltered and dialysed. Finally, the CS sodium salt is filtered and dried under vacuum to a residual humidity of below 10%.

The molecular dimensions of the CS obtained are then reduced by a depolymerisation process performed according to known radical depolymerisation (Volpi N. et al., Carb. Res., 279, 193-200, 1995) or acidic depolymerisation techniques, controlling the process so as to obtain the required molecular weight distribution.

Acidic depolymerisation is performed by resuspending the CS in water, acidifying the solution with the addition of HCl to a concentration of 1 M, and heating to 60°C.

The molecular weight of the oligosaccharides generated by depolymerisation is calculated by taking samples of the solution at short intervals, determining the molecular weight of the oligosaccharides by SEC-HPLC analysis carried out on two 5 µm Agilent Bio Series SEC^{©}5 (hydrophilic neutral polymeric monolayer) columns of 300 and 150 Å respectively, in series. The reaction is interrupted by neutralisation with NaOH or sodium bicarbonate, so that the pH is adjusted to 6-8 when the desired molecular mass values have been reached.

Alternatively, depolymerisation can be obtained by radical hydrolysis, controlling the final molecular weight of the resulting oligosaccharides as described previously.

The CS is resuspended in water and the pH is corrected to 7.5 by adding a 10% hydrochloric acid or sodium hydroxide solution, depending on whether the CS solution needs to be acidified or basified. A 9% solution of hydrogen peroxide (H₂O₂) is added to the solution maintained at 60°C. SEC-HPLC is performed as previously described to check whether the desired molecular weight has been reached. The reaction is interrupted by cooling the solution to room temperature (20-25°C) and lowering the pH to 6.0.

Table 4 shows the molecular weight values typical of an oligosaccharide analysed with SEC-HPLC during the reaction steps until the end of depolymerisation.

**Table 4**

| Time (minutes) | MWw (kDa) | Polydispersity Index | Relative MWw (% of initial value) |
|---|---|---|---|
| 0 | 77.3 | 0.2 | 100.0 |
| 60 | 73.6 | 0.2 | 95.3 |
| 120 | 81.9 | 0.2 | 106.0 |
| 180 | 76.3 | 0.3 | 98.7 |
| 330 | 45.7 | 0.4 | 59.1 |
| 390 | 39.7 | 0.4 | 51.4 |
| 510 | 28.6 | 0.5 | 37.0 |
| 660 | 25.5 | 0.6 | 33.0 |
| 780 | 20.5 | 0.6 | 26.5 |
| 840 | 18.7 | 0.6 | 24.2 |
| 900 | 18.1 | 0.6 | 23.4 |
| 1020 | 14.0 | 0.6 | 18.1 |
| 1200 | 10.2 | 0.6 | 13.2 |
| 1440 | 3.7 | 0.6 | 9.96 |

The C6S according to the invention can also be obtained by chemical sulphation according to the procedures previously indicated, using as substrate the low-molecular-weight fraction of polysaccharide K4 deriving from fermentation of *E. coli* strain O5:K4:H4. In this case, the culture broth is treated at the end of fermentation by heating at 80°C for 60 minutes to deactivate the micro-organism, and is then centrifuged and ultrafiltered as in EP 1304338; the resulting supernatant is then loaded onto a gel-filtration column and the fractions are collected, checking the uronic acid content of each one by known techniques. By combining the fractions that test positive to the uronic acid test, two separate pools can be isolated: a first pool containing high-molecular-weight K4 (40-70 kDa), corresponding to the known polysaccharide and quantitatively corresponding to 80% of the total saccharides, and a second pool, clearly separated from the first on the basis of the elution volume and containing low-molecular-weight K4, with low dispersion around the mean value, between 1500 and 6000 daltons. The identity of the oligosaccharides contained in said second low-molecular-weight pool with K4 is demonstrated by the simultaneous positive response to the uronic acid assay and digestibility with chondroitinase ABC, accompanied by the appearance of disaccharide units.

Said fraction of oligosaccharide K4, which quantitatively represents 20% of the total saccharides, is then subjected to the defructosylation and chemical sulphation process disclosed in PCT/EP2011/058297 until a CS with a final molecular weight in the 1000-5000 dalton range is obtained.

Alternatively, the low-molecular-weight biotechnological C6S can be obtained by a process similar to those previously described, involving sulphation of the low-molecular-weight fraction of the naturally defructosylated oligosaccharide K4-d recovered from fermentation of *E. coli* strain *DSM23644* described in WO 2012004063.

The low-molecular-weight C6S thus obtained was evaluated for efficacy in an experimental animal arthritis model (Adjuvant Arthritis: AA) in the rat, and the results obtained were compared with those relating to pharmaceutical grade natural CS of extractive origin used in the same experimental model (Bauerova K. et al., Osteoarthritis Cartilage 19, 1373, 2011) after daily oral treatment with 900 mg/kg.

AA was induced by a single intradermal injection of *Mycobacterium butyricum* in incomplete Freund's adjuvant. The study involved one group of healthy animals (HC), one group of untreated arthritic animals (AC) and two groups of arthritic animals treated with two different regimens. The first treatment regimen involved pre-treatment consisting of administration of 900 mg/kg of biotechnological C6S a day for 14 days before arthritis was induced, continuing for 28 days after the induction of AA (PT). The second treatment regimen involved the administration of 900 mg/kg of biotechnological C6S a day only during the 28 days after induction of AA (T).

The physiological increase in body weight of the rats was very low in the untreated arthritic animals (AC), amounting to about 40% of that of the healthy controls at the end of the study. Pre-treatment with biotechnological C6S (PT group) limited this reduction: the increase in body weight amounted to 73% of that of the healthy controls. The treatment alone (T) also proved effective in restoring body weight, though to a lesser extent (an increase of 54% compared with the healthy controls) (Fig. 1). This is attributable to the anti-inflammatory role of low-molecular-weight biotechnological C6S at systemic level. This effect on the increase in body weight of the animals is higher than that found in the study by Bauerova et al., conducted with a high-molecular-weight CS of bovine origin at the same dose (Bauerova K. Et al., Osteoarthritis Cartilage 19, 1373, 2011). This finding confirms the greater intestinal absorption of the biotechnological C6S according to the invention.

The severity of the arthritis was quantified on the basis of the increasing levels of swelling of the limbs (oedema); the oedema that developed in the hind paw was significantly reduced in the pre-treated animals (PT) (Fig. 2). Pre-treatment with biotechnological C6S significantly reduced oedema throughout the study compared with the untreated controls (Fig. 3).

The pre-treatment also proved effective in reducing the total arthritis score, a parameter which takes account of a set of clinical factors comprising periarticular erythema, developed oedema and the diameter of the scab at the adjuvant injection site at the base of the tail. The arthritis evaluation scale allocates a score between 6 and 31; the arthritis control group (AC) obtained a value of 23, whereas the PT group reached a value of 19, as against 12 for the healthy controls (HC) (Fig. 4). Moreover, the pre-treatment proved effective throughout the subacute phase, from day 1 to day 28 after induction of AA (Fig. 5). The treatment-only (T) group did not significantly influence the arthritis score during the study period.

The C6S according to the invention was also tested for its toxicological safety in animals and on cell cultures according to various protocols designed to assess its potential genotoxicity at cell level and acute oral toxicity in the rat. All the tests used were validated and conducted according to OECD guidelines for pharmaceutical products.

The biotechnological C6S was subjected to mutagenesis tests in bacterial cells (bacterial reverse mutation, Ref. OECD 471) which tested the ability of the product to induce the appearance of reverse mutants in auxotrophic strains of *E. coli* and *Salmonella typhimurium.* No significant increase in bacterial mutagenicity was observed.

The genotoxicity of biotechnological C6S was also examined in two other tests on eukaryotic cell cultures, namely the test for chromosome aberrations in Chinese hamster ovary cells *in vitro,* OECD Ref. 473) and a mutagenicity test on murine lymphoma cells (Mutation in L5178Y TK^{+/+} mouse lymphoma cells, Prot. OECD 476). No significant increase in genetic toxicity was found in the two studies cited up to the highest C6S concentration used (5000 µg/plate and 5000 µg/ml respectively).

Finally, acute toxicity after oral administration was examined in Sprague-Dawley rats up to the dose of 2000 mg/kg of body weight. After observation lasting 14 days after the administration, the rats did not show any clinical signs of suffering, and no mortality occurred. Moreover, the autopsy performed at the end of the study did not indicate any signs of toxicity in the tissues and organs examined.

For the proposed therapeutic or health uses, the C6S according to the invention will be used as the active ingredient of medicaments, diet supplements or food additives, possibly combined with other active ingredients such as glucosamine hydrochloride, glucosamine sulphate, N-acetylglucosamine, hyaluronic acid, amino acids, collagen, hydrolysed collagen, polyunsaturated fatty acids, keratin, dermatin, methylsulphonylmethane (MSM), folates, reduced folates, vitamins, group B vitamins, S-adenosylmethionine (SAMe), ascorbic acid or manganese ascorbate.

Examples of formulations according to the invention include capsules, soft gel capsules, tablets, drinks in liquid form, and powdered drinks to be reconstituted.

The doses of the C6S according to the invention will be between 100 and 3000 mg/day, preferably between 1000 and 2000 mg/day, and more preferably between 1250 and 1750 mg/day.

The invention will now be described in greater detail in the following examples.

### Example 1: induction of arthritis (Adjuvant Arthritis, AA) in rats, and treatment with low-molecular-weight biotechnological C6S

40 male Lewis rats weighing between 150 and 190 g were divided at random into four groups of 10 animals each, housed in polypropylene cages in an environment maintained at the temperature of 22 ± 2°C, and fed on a standard laboratory diet with unlimited access to water.

The experimental groups were as follows:
1) An untreated healthy control group (HC).
2) An untreated control group with adjuvant-induced arthritis (AC).
3) A group of arthritic rats orally treated with biotechnological C6S at the dose of 900 mg/day per kg of body weight for 28 days after induction of AA (days 0-28 of the experiment) (T).
4) A group orally pre-treated with biotechnological C6S at the dose of 900 mg/day per kg of body weight for 14 days preceding the induction of AA, and for the 28 days after induction of AA (days -14 to 28 of the experiment) (PT).

Arthritis was experimentally induced in the rats on day 0 by a single intradermal injection at the base of the tail of 1 ml of a mixture consisting of *Mycobacterium butyricum* inactivated by heat in incomplete Freund's adjuvant.

The C6S of the invention was dissolved in distilled water at the concentration of 20 mg/ml and administered orally as a single daily dose by gavage.

### Example 2: effects of biotechnological C6S on the assessment of AA in rats by monitoring body weight

The body weight of the rats was measured before induction of AA (day 0), on days 7, 14 and 21, and at the end of the treatment (day 28). The effect of the treatment on this parameter was evaluated by comparing the weight increases of the different groups during the treatment period.

The values found are reported in Table 5:

**Table 5**

| | **Change in body weight:** Δ**(dayₙ - day₀)** | | | | |
|---|---|---|---|---|---|
| **Day (dayₙ)** | **0** | **7** | **14** | **21** | **28** |
| Healthy Control (HC) | 0.0 | 98.19 | 120.93 | 135.37 | 148.33 |
| *SEM* | *0.0* | *1.76* | *2.01* | *1.99* | *2.47* |
| Arthritic Control (AC) | 0.0 | 74.73 | 76.77 | 51.93 | 57.57 |
| *SEM* | *0.0* | *4.06* | *7.02* | *6.05* | *5.71* |
| LMW-C6S Treatment (T) | 0.0 | 85.19 | 89.19 | 68.39 | 79.78 |
| *SEM* | *0.0* | *3.03* | *5.63* | *7.52* | *8.86* |
| LMW-C6S Pre-Treatment (PT) | 0.0 | 92.96 | 107.26 | 93.39 | 108.63 |
| *SEM* | *0.0* | *2.94* | *6.48* | *8.65* | *8.29* |

| | | | | | |
|---|---|---|---|---|---|
| SEM: Standard Error of the Mean | | | | | |

### Example 3: effects of biotechnological C6S on the assessment of AA in rats by monitoring the oedema developed

The oedema that developed as a consequence of arthritis was measured by observing the increase in volume of the hind paw with a caliper suitable for the measurement. The measurements were performed before the induction of AA (day 0) and on days 7, 14, 21 and 28 of the study.

The data were expressed as the percentage increase in oedema calculated with the following formula: [(Dayₙ/Day₀) x 100] - 100, Day₀ being the measurement on the initial day and Dayₙ the measurement on the day considered.

The values found are reported in Table 6:

**Table 6**

| | **Change in hind paw swelling: [(Dayₙ/Day₀) x 100] - 100 (%)** | | | | |
|---|---|---|---|---|---|
| **Day (dayₙ)** | **0** | **7** | **14** | **21** | **28** |
| Healthy Control (HC) | *0.0* | 17.6 | 19.3 | 24.8 | 29.1 |
| *SEM* | *0.0* | *1.5* | *1.4* | *1.8* | *2.0* |
| Arthritic Control (AC) | *0.0* | 8.6 | 31.0 | 56.7 | 59.3 |
| *SEM* | *0.0* | *1.2* | *4.6* | *6.5* | *6.1* |
| LMW-C6S Treatment (T) | *0.0* | 13.1 | 34.5 | 62.8 | 61.4 |
| *SEM* | *0.0* | *1.0* | *6.4* | *8.1* | *7.1* |
| LMW-C6S Pre-Treatment (PT) | *0.0* | 15.4 | 26.7 | 46.5 | 49.7 |
| *SEM* | *0.0* | *1.5* | *4.9* | *6.9* | *7.1* |

| | | | | | |
|---|---|---|---|---|---|
| SEM: Standard Error of the Mean | | | | | |

### Example 4: effects of biotechnological C6S on the assessment of AA in rats by monitoring the arthritis score

The arthritis score was evaluated by allocating a score to the observation of paw joint swelling (oedema), the extent of periarticular erythema and the diameter of the scab at the adjuvant injection site at the base of the tail. The arthritis score or arthrogram was measured as the sum total of oedema (in ml, score 1 to 8), plus the diameter of the forepaw (in mm, max score 1 to 5), plus the diameter of the scab at the site of application of *Mycobacterium butyricum* measured parallel to the spinal column (in mm, max score 1 to 5), for each animal.

The values found are reported in Table 7:

**Table 7**

| | **Arthritis score** | | | | |
|---|---|---|---|---|---|
| **Day (dayₙ)** | **0** | **7** | **14** | **21** | **28** |
| Healthy Control (HC) | 10.0 | 10.0 | 10.2 | 11.4 | 12.0 |
| *SEM* | *0.0* | *0.0* | *0.1* | *0.3* | *0.0* |
| Arthritic Control (AC) | 10.0 | 11.0 | 16.9 | 22.4 | 23.2 |
| *SEM* | *0.0* | *0.4* | *1.2* | *1.4* | *1.3* |
| LMW-C6S Treatment (T) | 10.0 | 10.0 | 18.1 | 22.7 | 23.0 |
| *SEM* | *0.0* | *0.0* | *1.7* | *1.9* | *1.3* |
| LMW-C6S Pre-Treatment (PT) | 10.0 | 10.1 | 13.1 | 15.8 | 19.0 |
| *SEM* | *0.0* | *0.1* | *0.8* | *1.3* | *1.7* |

| | | | | | |
|---|---|---|---|---|---|
| SEM: Standard Error of the Mean | | | | | |

## Claims

1. A chondroitin sulphate having a molecular weight ranging from 1000 to 5000 daltons having anti-inflammatory and anti-arthritic biological activity **characterised by** a percentage of disaccharide 6-monosulphate equal to or greater than 65%, a percentage of disaccharide 4-monosulphate < 1%, a percentage of disaccharide 2,6-disulphate lower than or equal to 20%, a percentage of disaccharide 4,6-disulphate < 5%, a percentage of disaccharide 2,4-disulphate < 1%, a percentage of non-sulphated disaccharide < 15%, and a charge density value ranging from 1 to 1.25.

2. A chondroitin sulphate according to claim 1 obtained by chemical sulphation and subsequent acid or radical depolymerisation of the capsular polysaccharide K4 of *E. coli* after removal of the fructose residues by means of hydrolysis.

3. A chondroitin sulphate according to claim 1 obtained by chemical sulphation of the low-molecular-weight natural fraction of the capsular polysaccharide K4 of *E. coli* carried out after removal of the fructose residues by means of hydrolysis.

4. A chondroitin sulphate according to claim 1 obtained by chemical sulphation and subsequent acid or radical depolymerisation of the capsular polysaccharide originally free from fructose residues (K4-d), produced by the *E. coli* strain *DSM23644.*

5. A chondroitin sulphate according to claim 1 obtained by chemical sulphation of the low-molecular-weight fraction of capsular polysaccharide originally free from fructose residues (K4-d) produced by the *E. coli* strain *DSM23644.*

6. Chondroitin sulphate of claims 1-5 for use in the prevention and treatment of acute or chronic inflammatory conditions and/or for the preservation of musculoskeletal health in humans and animals.

7. Chondroitin sulphate for use according to claim 6 wherein the inflammatory condition is osteoarthritis.

8. Compositions comprising the chondroitin sulphate of claims 1-5 as active ingredient, in combination with pharmaceutically and nutraceutically acceptable excipients and optionally with other active ingredients.

9. Compositions according to claim 8 wherein the other active ingredients are selected from glucosamine hydrochloride, glucosamine sulphate, N-acetyl-glucosamine, hyaluronic acid, amino acids, collagen, hydrolysed collagen, polyunsaturated fatty acids, keratin, dermatin, methylsulphonylmethane (MSM), folates, reduced folates, vitamins, group B vitamins, S-adenosylmethionine (SAMe), ascorbic acid or manganese ascorbate.

10. Compositions according to claim 8 or 9 in the form of capsules, soft gel capsules, tablets, drinks in liquid form or drinks in powder form to be dissolved.

## Patentansprüche

1. Ein Chondroitinsulfat, dessen Molekulargewicht im Bereich von 1000 bis 5000 Dalton liegt, mit entzündungshemmender und anti-arthritischer biologischer Aktivität, charakterisiert durch einen Anteil von Disaccharid-6-monosulfat gleich oder größer als 65%, einen Anteil von Disaccharid-4-monosulfat < 1%, einen Anteil von Disaccharid-2,6-disulfat niedriger als oder gleich 20%, einen Anteil von Disaccharid-4,6-disulfat < 5%, einen Anteil von Disaccharid-2,4-disulfat < 1%, einen Anteil nicht-sulfatierten Disaccharids < 15%, und einen Ladungsdichtewert von 1 bis 1.25.

2. Ein Chondroitinsulfat gemäß Anspruch 1, erhalten durch chemische Sulfatierung und nachfolgende saure oder radikalische Depolymerisation des kapsulären Polysaccharids K4 von *E. coli* nach der Entfernung der Fructose-Reste mittels Hydrolyse.

3. Ein Chondroitinsulfat gemäß Anspruch 1, erhalten durch chemische Sulfatierung der natürlichen Fraktion mit niedrigem Molekulargewicht des kapsulären Polysaccharids K4 von *E. coli,* durchgeführt nach der Entfernung der Fructose-Reste mittels Hydrolyse.

4. Ein Chondroitinsulfat gemäß Anspruch 1, erhalten durch chemische Sulfatierung und nachfolgende saure oder radikalische Depolymerisation des ursprünglich von Fructose-Resten freien kapsulären Polysaccharids (K4-d), hergestellt von dem *E. coli*-Stamm DSM23644.

5. Ein Chondroitinsulfat gemäß Anspruch 1, erhalten durch chemische Sulfatierung der Fraktion mit niedrigem Molekulargewicht des ursprünglich von Fructose-Resten freien Polysaccharids (K4-d), hergestellt von dem *E. coli-*Stamm DSM23644.

6. Chondroitinsulfat der Ansprüche 1-5 zur Anwendung in der Prävention und Behandlung akuter oder chronischer entzündlicher Zustände und/oder zur Bewahrung der muskuloskeletalen Gesundheit bei Menschen und Tieren.

7. Chondroitinsulfat zur Anwendung gemäß Anspruch 6, worin der entzündliche Zustand Osteoarthritis ist.

8. Zusammensetzungen, umfassend das Chondroitinsulfat der Ansprüche 1-5 als wirksamen Inhaltsstoff, in Kombination mit pharmazeutisch und nutrazeutisch annehmbaren Hilfsstoffen und optional mit anderen aktiven Inhaltsstoffen.

9. Zusammensetzungen nach Anspruch 8, worin die anderen aktiven Inhaltsstoffe ausgewählt sind aus Glucosaminhydrochlorid, Glucosaminsulfat, N-Acetylglucosamin, Hyaluronsäure, Aminosäuren, Kollagen, hydrolysiertem Kollagen, mehrfach ungesättigten Fettsäuren, Keratin, Dermatin, Methylsulfonylmethan (MSM), Folaten, reduzierten Folaten, Vitaminen, Vitaminen der B-Gruppe, S-Adenosylmethionin (SAMe), Ascorbinsäure oder Manganascorbat.

10. Zusammensetzungen nach Anspruch 8 oder 9 in Form von Kapseln, weichen Gelkapseln, Tabletten, Getränken in flüssiger Form oder Getränken in Form von Pulver zum Auflösen.

## Revendications

1. Sulfate de chondroïtine dont le poids moléculaire se situe dans la plage de 1000 à 5000 Daltons, possédant une activité biologique antiinflammatoire et antiarthritique, **caractérisé par** un pourcentage du 6-monosulfate de disaccharide égal ou supérieur à 65 %, un pourcentage du 4-monosulfate de disaccharide inférieur à 1 %, un pourcentage du 2,6-disulfate de disaccharide inférieur ou égal à 20 %, un pourcentage du 4,6-disulfate de disaccharide inférieur à 5 %, un pourcentage du 2,4-disulfate de disaccharide inférieur à 1 %, un pourcentage de disaccharide non sulfaté inférieur à 15 %, et par une valeur de densité de charge qui se situe dans la plage de 1 à 1,25.

2. Sulfate de chondroïtine selon la revendication 1, que l'on obtient par sulfatation chimique et dépolymérisation ultérieure par voie acide ou par voie radicalaire, du polysaccharide capsulaire K4 de *E*. *coli* après élimination des résidus de fructose au moyen d'une hydrolyse.

3. Sulfate de chondroïtine selon la revendication 1, que l'on obtient par sulfatation chimique de la fraction naturelle de bas poids moléculaire du polysaccharide capsulaire K4 de *E. coli,* mise en oeuvre après élimination des résidus de fructose au moyen d'une hydrolyse.

4. Sulfate de chondroïtine selon la revendication 1, que l'on obtient par sulfatation chimique et dépolymérisation ultérieure par voie acide ou par voie radicalaire, du polysaccharide capsulaire exempt à l'origine de résidus de fructose (K4-d) produit par la souche *DSM23644* de *E. coli.*

5. Sulfate de chondroïtine selon la revendication 1, que l'on obtient par sulfatation chimique de la fraction de bas poids moléculaire du polysaccharide capsulaire exempt à l'origine de résidus de fructose (K4-d) produit par la souche *DSM23644* de *E. coli.*

6. Sulfate de chondroïtine selon les revendications 1 à 5, pour son utilisation dans la prévention et le traitement d'affections inflammatoires aiguës ou chroniques et/ou pour la préservation de la santé musculosquelettique chez les êtres humains et les animaux.

7. Sulfate de chondroïtine pour son utilisation selon la revendication 6, dans lequel l'affection inflammatoire est l'ostéoarthrite.

8. Compositions comprenant le sulfate de chondroïtine selon les revendications 1 à 5 à titre d'ingrédient actif, en combinaison avec des excipients pharmaceutiquement et nutraceutiquement acceptables et de manière facultative avec d'autres ingrédients actifs.

9. Compositions selon la revendication 8, dans lesquelles les autres ingrédients actifs sont choisis parmi le chlorhydrate de glucosamine, le sulfate de glucosamine, la N-acétyl-glucosamine, l'acide hyaluronique, des acides aminés, du collagène, du collagène hydrolysé, des acides gras polyinsaturés, la kératine, la dermatine, le méthyl-sulfonylméthane (MSM), des folates, des folates réduits, des vitamines, des vitamines du groupe B, la S-adénosylméthionine (SAMe), l'acide ascorbique ou l'ascorbate de manganèse.

10. Composition selon la revendication 8 ou 9 sous la forme de capsules, de capsules de gélatine molle, de comprimés, de boissons sous forme liquide ou de boissons sous forme pulvérulente qui doivent être dissoutes.
